# DEMANDE DE BREVET EUROPEEN

(11) **EP 1 559 414 A1**
(43) Date de publication de la demande: **03.08.2005**
(21) Numéro de dépôt: 05290141.0
(22) Date de dépôt: 21.01.2005
(51) Int. Cl.: A61K 7/42

(54) **Utilisation d'une composition obtenue à partir de fluide sous pression et d'agents protecteurs pour la protection des fibres kératiniques**

(30) Priorité: 29.01.2004 FR 0400851
(71) Demandeur: L'OREAL, 75008 Paris (FR)
(72) Inventeur: De La Mettrie, Roland, 78110 Le Vesinet (FR)
(74) Mandataire: Casalonga, Axel

(57) **Abrégé**

L'invention concerne l'utilisation cosmétique d'une composition obtenue par percolation d'un fluide sous une pression d'au moins 3 bars au travers d'au moins un agent protecteur des fibres kératiniques, sous forme solide ou pâteuse, pour la protection des fibres kératiniques contre les agressions extérieures.

## Description

La présente invention a pour objet l'utilisation de compositions cosmétiques obtenues à partir de fluide sous pressions et d'agents protecteurs pour la protection des fibres kératiniques contre les agressions extérieures. Elle a également pour objet un procédé de préparation d'une composition cosmétique de traitement des fibres kératiniques.

Il est bien connu que les fibres kératiniques telles que les cheveux, les cils ou les sourcils sont sensibilisées ou fragilisées à des degrés divers par l'action des agents atmosphériques et de la lumière, du graissage, du chlore, ainsi que par l'action répétée de différents traitements plus ou moins agressifs tels que les permanentes, le défrisage, la teinture, la décoloration, les lavages et autres. Les cheveux deviennent alors rêches au toucher, sont difficiles à démêler et à coiffer. En outre, les propriétés mécaniques des fibres kératiniques comme la résistance à la traction, la charge de rupture et l'élasticité, sont à la longue altérées.

On peut alors appliquer sur ces fibres kératiniques des compositions de traitement cosmétique comprenant des agents protecteurs des fibres kératiniques tels que des filtres solaires ou des flavonoïdes.

Par « agents protecteurs des fibres kératiniques », on entend des agents qui protègent les fibres kératiniques contre les agressions extérieures, en particulier celles décrites ci-dessus.

Par « protection des fibres kératiniques contre les agressions extérieures », on entend la diminution de l'altération des propriétés physico-chimiques des fibres kératiniques, et notamment la résistance à la traction, la charge de rupture et l'élasticité, sous l'action des agents extérieurs. Cet effet cosmétique se traduit par un meilleur aspect des fibres.

Cependant, les compositions de traitement cosmétique contenant de tels agents protecteurs sont généralement des compositions aqueuses dans lesquelles lesdits agents doivent être solubilisés. Le manque de solubilité de ces composés diminue le pouvoir traitant de ces compositions. En outre, ce critère de solubilité réduit le nombre d'agents protecteurs insolubles dans l'eau que l'on peut utiliser pour le traitement cosmétique des fibres kératiniques. Ceci est particulièrement le cas des composés à point de fusion élevé.

La Demanderesse a découvert de manière surprenante qu'en utilisant un procédé de préparation d'une composition de traitement cosmétique des fibres kératiniques particulier, on pouvait obtenir en un temps très court, inférieur à 2 minutes, des compositions plus ou moins concentrées en agent(s) protecteur(s) selon le besoin, notamment sans conservateur, permettant de surmonter les problèmes de solubilité exposés ci-dessus.

Ce procédé est mis en oeuvre simplement. On fait passer un fluide sous pression, dont la température est de préférence supérieure
ou égale à 30°C, et est encore plus préférentiellement allant de 30 à 150°C, pendant un laps de temps très court, inférieur à une minute, à travers au moins un agent protecteur sous forme solide ou pâteuse, de préférence sous forme solide, et encore de préférence sous forme pulvérulente.

Il permet d'utiliser sous forme anhydre des agents protecteurs instables dans des compositions aqueuses, soit parce qu'ils réagissent avec l'eau, soit parce qu'ils réagissent en solution aqueuse avec des composés qui ne réagissent pas avec eux dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température cosmétiquement acceptable, de préférence inférieure à 60°C. La composition peut être utilisée jusqu'à une semaine après sa préparation, selon la vitesse de dégradation de l'agent protecteur utilisé.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les agents protecteurs peuvent être conditionnés dans un dispositif prêt-à-l'emploi, et il n'est pas nécessaire de déterminer au préalable les concentrations desdits agents en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage supplémentaire de ce procédé de préparation est l'obtention de compositions conférant de bonnes propriétés cosmétiques. En particulier, les fibres kératiniques traitées avec une composition obtenue par le procédé selon l'invention présentent de bonnes propriétés de résistances aux agressions extérieures, et notamment contre la pollution, le graissage, les radiations UV, la lumière, les radicaux libres et le chlore.

L'invention a donc pour objet l'utilisation cosmétique d'une composition obtenue par percolation d'un fluide sous une pression d'au moins 3 bars au travers d'au moins un agent protecteur des fibres kératiniques, sous forme solide ou pâteuse, pour la protection des fibres kératiniques contre les agressions extérieures.

L'invention a également pour objet un procédé de préparation d'une composition de traitement cosmétique des fibres kératiniques comprenant une étape de percolation d'un fluide sous une pression d'au moins 3 bars au travers d'au moins un filtre solaire, sous forme solide ou pâteuse.

Un autre objet de l'invention est une composition susceptible d'être obtenue par le procédé selon l'invention.

L'invention a enfin pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

De préférence, les fibres kératiniques sont les cheveux.

L'utilisation de la composition obtenue par percolation d'un fluide sous une pression d'au moins 3 bars au travers d'au moins un agent protecteur des fibres kératiniques, sous forme solide ou pâteuse, permet de protéger les fibres kératiniques contre les agressions extérieures, et notamment contre la pollution, le graissage, les radiations UV, la lumière, les radicaux libres et le chlore, en particulier contre les radiations UV.

Selon l'invention, le procédé de préparation d'une composition de traitement cosmétique des fibres kératiniques comprend une étape de percolation d'un fluide de préférence à une température supérieure ou égale à 30°C, mieux encore allant de 30 à 150°C, mieux encore de 40°C à 120°C, sous une pression d'au moins 3 bars (3.10⁵ Pa) au travers d'au moins un agent protecteur des matières kératiniques, sous forme solide ou pâteuse.

La percolation est un mouvement de fluide à travers un milieu poreux saturé permettant le passage du fluide, sous l'action ou l'effet de la pression.

Le fluide peut être constitué par de la vapeur d'eau éventuellement accompagnée d'eau liquide, ou par un ou plusieurs solvants liquides et/ou gazeux cosmétiquement acceptables, notamment organiques, ou encore par un mélange de vapeur d'eau éventuellement accompagnée d'eau liquide, et d'un ou plusieurs solvants liquides et/ou gazeux cosmétiquement acceptables. De préférence, le fluide comprend au moins de la vapeur d'eau pouvant être accompagnée d'eau liquide, et encore plus préférentiellement il est de la vapeur d'eau pouvant être accompagnée d'eau liquide.

A titre de solvant organique, on peut par exemple citer les alcools inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Par « composé insoluble dans l'eau », on entend tout composé qui, à une concentration supérieure ou égale à 0,1% en poids dans l'eau à 25°C, ne forme pas à l'oeil nu une solution isotrope transparente.

L'agent protecteur est sous forme solide ou sous forme pâteuse, de préférence sous forme solide, et encore plus préférentiellement sous forme pulvérulente.

Par « forme pâteuse » au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Le procédé de la présente invention peut être mis en oeuvre à partir d'un dispositif classique permettant de générer un fluide sous pression, à une température de préférence supérieure ou égale à 30°C, et encore plus préférentiellement allant de 30 à 150°C. Un tel dispositif comprend une chambre résistant à la pression, équipée d'un bloc thermique, ainsi qu'un circuit d'acheminement du fluide produit vers l'agent protecteur.

Selon un autre mode de réalisation, le dispositif comprend un réservoir de liquide(s) ainsi qu'une pompe permettant l'acheminement du ou des liquides vers la chambre.

Le liquide contenu dans le réservoir est soit de l'eau, soit un solvant cosmétiquement acceptable ou un mélange de plusieurs solvants cosmétiquement acceptables, ou encore un mélange d'eau et d'un ou plusieurs solvants cosmétiquement acceptables. De préférence, le liquide comprend au moins de l'eau, et encore plus préférentiellement il est de l'eau.

Un dispositif particulièrement utile pour la mise en oeuvre du procédé de la présente invention est une machine à café du type « expresso ». De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure ou égale à 30°C, et de préférence allant de 30 à 150°C, sous une pression comprise entre 3 et 30 bars, ou d'au moins 4 bars, de préférence supérieure à 10 bars et tout particulièrement comprise entre 10 et 30 bars.

Selon un mode de réalisation particulier de l'invention, l'étape de percolation est mise en oeuvre avec de la vapeur d'eau sous une pression d'au moins 4 bars, de préférence supérieure à 10 bars et tout particulièrement comprise entre 10 et 30 bars.

Le ou les agents protecteurs, sous forme solide ou pâteuse, peuvent être utilisés directement dans le dispositif générant le fluide sous pression dans un récipient destiné à cet usage. Ils peuvent également être conditionnés dans un dispositif de conditionnement d'une composition cosmétique particulier comprenant un logement fermé délimité par au moins une paroi au moins en partie perméable à un fluide sous pression d'au moins 3 bars, la composition contenant au moins un agent protecteur des fibres kératiniques sous forme solide ou pâteuse. De tels dispositifs sont par exemple décrits dans les demandes de brevets WO 00/56629, EP512470 ou WO 9903753.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple, en cellulose, en métal tel que l'aluminium ou en composite. De tels dispositifs sont par exemple décrits dans les demandes de brevets WO 00/56629, EP512470 ou WO 9903753.

Les agents protecteurs pouvant être utilisés dans le procédé selon l'invention peuvent être choisis parmi les filtres solaires, les antioxydants, les agents anti-pollution et les agents anti-radicalaires. On utilise de préférence les filtres solaires.

Les filtres solaires utilisés dans l'invention sont notamment choisis parmi les anthranilates ; les dérivés cinnamiques ; les dérivés de dibenzoylméthane ; les dérivés salicyliques ; les dérivés du camphre ; les dérivés de triazine tels que ceux décrits dans les demandes de brevet US 4367390, EP863145, EP517104, EP570838, EP796851, EP775698, EP878469, EP933376, EP507692, EP507691EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés de bis-benzoazolyle tels que décrits dans les brevets EP669323 et US 2,463,264 ; les dérivés de l'acide p-aminobenzoïque (PABA) ; les dérivés de méthylène bis-(hydroxyphényl benzotriazole) tels que décrits dans les demandes US 5,237,0,71, US 5,166,355, GB2303549, DE 19726184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO93/04665 ; les dimères dérivés d'α-alkylstyrène tels que ceux décrits dans la demande de brevet DE19855649 ; les 4,4-diarylbutadiènes tels que ceux décrits dans les demandes EP0967200, DE19746654, DE19755649, EP-A-1008586, EP1133980 et EP1133981 et leurs mélanges.

Comme exemple de filtres organiques, on peut citer ceux désignés ci-dessous sous leur nom INCI :
Dérivés de l'acide para-aminobenzoique :
   PABA,
   Ethyl PABA,
   Ethyl Dihydroxypropyl PABA,
   Glyceryl PABA,
Dérivés salicyliques :
   Dipropyleneglycol Salicylate vendu sous le nom « DIPSAL » par SCHER,
   TEA Salicylate, vendu sous le nom « NEO HELIOPAN TS » par HAARMANN et REIMER,
Dérivés du dibenzoylméthane :
   Butyl Methoxydibenzoylmethane, vendu notamment sous le nom commercial « PARSOL 1789 » par ROCHE VITAMINS,
   Isopropyl Dibenzoylméthane,
Dérivés cinnamiques :
   Ethylhexyl Methoxycinnamate vendu notamment sous le nom commercial « PARSOL MCX » par ROCHE VITAMINS,
   Isopropyl Methoxy cinnamate,
   Isoamyl Methoxy cinnamate vendu sous le nom commercial « NEO HELIOPAN E 1000 » par HAARMANN et REIMER,
   DEA Methoxycinnamate, Diisopropyl Methylcinnamate,
   Glyceryl Ethylhexanoate Dimethoxycinnamate
Dérivés de β,β-dinhénylacrylate :
   Ethocrylene, vendu notamment sous le nom commercial « UVINUL N35 » par BASF,
Dérivés de la benzophénone :
   Benzophénone-1 vendu sous le nom commercial « UVINUL 400 » par BASF,
   Benzophénone-2 vendu sous le nom commercial « UVINUL D50 » par BASF,
   Benzophénone-3 ou Oxybenzone vendu sous le nom commercial « UVINUL M40 » par BASF ,
   Benzophénone-4 vendu sous le nom commercial « UVINUL MS40 » par BASF,
   Benzophénone-5,
   Benzophénone-6 vendu sous le nom commercial « HELISORB 11 » par NORQUAY,
   Benzophénone-8 vendu sous le nom commercial « Spectra-Sorb UV-24 » par American Cyanamid,
   Benzophénone-9 vendu sous le nom commercial « UVINUL DS-49 » par BASF,
   Benzophénone-12,
   2-(4-diethylamino-2-hyroxybenzoyl)-benzoate de n-hexyle vendu sous le nom commercial « UVINUL A PLUS » par BASF,
Dérivés du benzylidène camphre :
   3-Benzylidene camphor fabriqué sous le nom « MEXORYL SD » par CHIMEX,
   4-Methylbenzylidene camphor vendu sous le nom « EUSOLEX 6300 » par MERCK,
   Benzylidene Camphor Sulfonic Acid fabriqué sous le nom « MEXORYL SO » par CHIMEX,
   Camphor Benzalkonium Methosulfate fabriqué sous le nom « MEXORYL SO » par CHIMEX,
   Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phenyl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial « NEO HELIOPAN AP » par HAARMANN et REIMER,
Dérivés de la triazine :
   Anisotriazine vendu sous le nom commercial « TINOSORB S » par CIBA GEIGY,
   Ethylhexyl triazone vendu notamment sous le nom commercial « UVINUL T150 » par BASF,
   Diethylhexyl Butamido Triazone vendu sous le nom commercial « UVASORB HEB » par SIGMA 3V,
Dérivés du phenyl benzotriazole :
Drometrizole Trisiloxane vendu sous le nom « Silatrizole » par RHODIA CHIMIE
Methylène bis-Benzotriazolyl Tetramethylbutylphénol, vendu sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial « NEO HELIOPAN MA » par HAARMANN et REIMER,
Dérivés d'imdazolines ;
   Ethylhexyl Dimethoxybenzylidene Dioxoimidazoline Propionate,
Dérivés du benzalmalonate :
   Polyorganosiloxanes à fonction benzalmalonate tels que le Polysilicone-15 vendu sous la dénomination commerciale « PARSOL SLX » par ROCHE VITAMINS.
Dérivés de 4,4-diarylbutadiène :
   -1,1-dicarboxy(2,2'-diméthyl-propyl)-4,4-diphénylbutadiène
Dérivés de benzoxazole :
   2,4-bis-[5-1(dimethylpropyl)benzoxazol-2-yl-(4-phenyl)-imino]-6-(2-ethylhexyl)-imino-1,3,5-triazine vendu sous le nom UVASORB K2A par SIGMA 3V ;
   et leurs mélanges.

Les filtres organiques UV préférentiels sont choisis parmi :
Ethylhexyl Methoxycinnamate,
Butyl Methoxydibenzoylmethane,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle,
4-Methylbenzylidene camphor,
Terephtalylidene Dicamphor Sulfonic Acid,
Disodium Phenyl Dibenzimidazole Tetra-sulfonate,
Anisotriazine,
Ethylhexyl triazone,
Diethylhexyl Butamido Triazone,
Methylene bis-Benzotriazolyl Tetramethylbutylphénol,
Drometrizole Trisiloxane,
Polysilicone-15,
1,1-dicarboxy (2,2'-diméthyl-propyl)-4,4-diphénylbutadiène,
2,4-bis[5-1(diméthylpropyl)benzoxazol-2-yl-(4-phenyl)-imino]-6-(2-ethylhexyl)-imino-1,3,5-triazine,
et leurs mélanges.

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone,utilisables dans la composition selon l'invention, on peut citer en particulier la vitamine C et ses dérivés dont le glucoside d'ascorbyle ; les phénols et polyphénols non colorants, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol : les dérivés d'acides aminés soufrés, en particulier la S carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles non colorants, en particulier l'indol-3-carbinol ; les extraits de thé en particulier de thé vert, les extraits de Jacinthe d'eau ou eichhornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichhornia crassipes) ; et la fraction hydrosoluble de maïs commercialisée par la société SOLABIA sous la dénomination commerciale Phytovityl®.

Les agents anti-radicalaires ou antioxydants utilisables dans la composition selon l'invention comprennent, outre certains agents anti-pollution mentionnés précédemment, la vitamine E et ses dérivés tels que l'acétate de tocophéryle ; les flavonoïdes ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; la mélatonine, les réductones et leurs dérivés dont l'acide érythorbique, l'acide ascorbique et le palmitate d'ascorbyle, le sulfite de sodium.

Comme flavonoïdes, on peut citer par exemple les isoflavonoïdes qui constituent une sous-classe des flavonoïdes, formés d'un squelette 3-phényl chromane qui peut comporter des substituants variés et différents niveaux d'oxydation. Le terme « isoflavonoïde » regroupe plusieurs classes de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromes, les α-méthyldeoxybenzoines, les 2-arylbenzofuranes, et leurs mélanges. A cet égard, on se reportera avantageusement pour une revue complète sur les isoflavonoïdes, leurs méthodes d'analyse et leurs sources, au chapitre 5 « Isoflavonoïds » écrit par P.M. Dewick, dans The Flavonoïds, Harbone éditeur, pp. 125-157 (1988).

Les isoflavonoïdes peuvent être d'origine naturelle ou synthétique. Par « origine naturelle », on entend l'isoflavonoïde à l'état pur ou en solution à différentes concentrations, obtenu par différents procédés d'extraction à partir d'un élément, généralement une plante, d'origine naturelle. Par « origine synthétique, on entend l'isoflavonoïde à l'état pur ou en solution à différentes concentration, obtenu par synthèse chimique.

Parmi les bioflavonoïdes, on peut citer les bioflavonoïdes de citrus. Les bioflavonoïdes de citrus sont présents dans les écorces de citrus tels que le citron, l'orange, la mandarine ou le pamplemousse. Ils sont connus également pour leur aptitude à maintenir en bon état les vaisseaux sanguins par diminution de la fragilité et de la perméabilité des vaisseaux capillaires (The Merck Index; 1989; page 1243).

Les bioflavonoïdes utilisables dans le cadre de la présente invention peuvent être choisis parmi les composés de formule (I) : 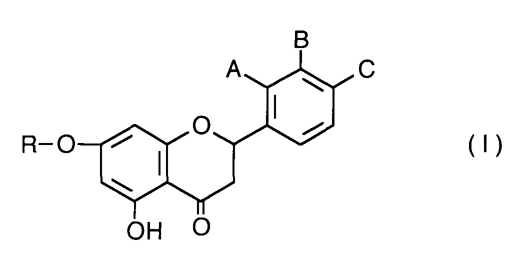 dans laquelle
- R représente un radical d'un sucre
- A représente un atome d'hydrogène ou un radical alcoxy ayant environ de 1 à 4 atomes de carbone,
- B représente un atome d'hydrogène ou un radical hydroxy ou alcoxy ayant environ de 1 à 4 atomes de carbone,
- C représente un atome d'hydrogène ou un radical hydroxy ou alcoxy ayant environ de 1 à 4 atomes de carbone,
ou de formule (II) : 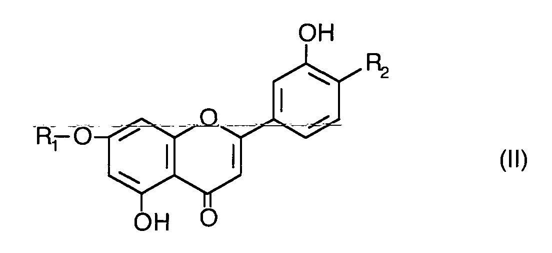 dans laquelle R₁ désigne un radical 6-O-(6-déoxy-α-L- mannopyranosyl)-β-D-glucopyranosyl et R₂ désigne un radical alcoxy ayant environ de 1 à 4 atomes de carbone.

Les bioflavonoïdes tel que définis par les formules (I) ou (II) ci-dessus sont des agents de traitement visant à la protection ou à l'amélioration des propriétés physiques, en particulier des propriétés mécaniques et/ou des propriétés cosmétiques des phanères kératiniques (les cheveux, les cils, les sourcils et les ongles).

Les propriétés mécaniques améliorées sont plus particulièrement la résistance à la traction, la charge de rupture ou l'élasticité.

Les propriétés cosmétiques sont, en particulier, le démêlage et/ou le coiffage et/ou la douceur.

Dans le cadre de la présente invention, A représente, de préférence, un atome d'hydrogène ou un radical méthoxy, B et C représentent, de préférence et indépendemment l'un de l'autre, un atome d'hydrogène ou un radical hydroxy ou méthoxy, R₂ représente de préférence un radical méthoxy. R représente, de préférence, un reste choisi parmi les groupements 6-O-(6-déoxy-α-L-manno-pyranosyl)-β-D-glucopyranosyl, 2-O-(6-déoxy-α-L-mannopyranosyl)-β-D-glucopy-ranosyl et 6-déoxy-α-L-mannopyranosyl.

Les composés de formule (I) encore plus particulièrement préférés sont choisis parmi la naringine, la néohespéridine, l'hespéridine et l'ériodictine.

Les composés de formule (I) et (II) sont des bioflavonoïdes qui sont extraits de plantes et peuvent être également obtenus selon les procédés décrits dans "The Flavonoïds" Harbone J.B., Mabry T.J., Helga Mabry, 1975.

Les bioflavonoïdes pouvant être utilisés selon l'invention possèdent une bonne affinité vis à vis des phanères kératiniques. Ils renforcent les propriétés physiques des phanères kératiniques, notamment contre la dégradation par la lumière. Ils préservent les propriétés mécaniques des phanères kératiniques, et notamment leur résistance à la traction, leur élasticité, leur vitesse de gonflement dans un milieu aqueux. Les cheveux ainsi traités présentent de bonnes propriétés cosmétiques notamment au niveau de leur facilité à être démêlés.

Les plantes ou extraits de plantes utilisés peuvent être soumis avant la percolation à un traitement tel qu'une torréfaction, un cryobroyage, ou une lyophilisation.

Les composés de formule (I) et (II) sont présents dans des extraits d'agrumes et en particulier de citron. De tels extraits sont notamment commercialisés par la société INTERCHEMICAL sous les dénominations "CITRUS BIOFLAVONOÏD COMPLEX 45%", "LEMON BIOFLAVONOÏD COMPLEX 50%", "GRAPEFRUIT BIOFLAVONOÏD COMPLEX 25% et "ORANGE BIOFLAVONOÏD COMPLEX 25%".

Le ou les agents protecteurs selon l'invention peuvent être mis en oeuvre en mélange avec un ou plusieurs adjuvants solides ou pâteux, et de préférence pulvérulents. Les adjuvants peuvent être choisis parmi les argiles, les sels, les agents tensioactifs anioniques, cationiques, non-ioniques, amphotères, zwittérioniques, les épaississants naturels ou de synthèse, les billes de verre, la silice, le nylon, l'alumine, le dioxyde de titane, les zéolithes, le polyméthylméthacrylate (PMMA), le chitosane, la maltodextrine, la cyclodextrine, les mono ou disaccharides comme le glucose, le saccharose, le sorbitol ou le fructose, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, l'amidon, éventuellement modifié, les grains de maïs, les gommes de polydiméthylsiloxane, le polyacrylamide, l'hydroxyapatite poreux. la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, les particules de poly(chlorure de vinylidène/acrylonitrile), notamment celles commercialisées sous la dénomination générale d' « Expancel® » par la société AKZO NOBEL sous les références particulières « Expancel® WE » ou « DE » Expancels, et leurs mélanges. A partir du procédé de préparation de l'invention, on obtient une composition de traitement cosmétique qui peut être mise en oeuvre soit directement soit après addition de différents composants.

Lorsqu'un ou plusieurs adjuvants sont présents, le ou les agents protecteurs de l'invention sont présents de préférence en une quantité allant de 0,5 à 99% en poids, mieux encore de 1 à 80 % en poids, et encore plus préférentiellement de 2 à 60 % en poids par rapport au poids total agent(s) protecteur(s) et adjuvants.

La composition de traitement cosmétique des matières kératiniques obtenue selon le procédé de l'invention contient outre le ou les agents protecteurs et le(s) composant(s) du fluide, à savoir l'eau et/ou le(s) solvant(s) cosmétiquement acceptable(s), éventuellement tout ou partie du ou des adjuvants présents dans le mélange solide ou pâteux.

A partir du procédé de préparation de l'invention, on obtient une composition de traitement cosmétique des fibres kératiniques qui peut être appliquée directement sur les fibres kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des fibres kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

La quantité du ou des agents protecteurs présents dans la composition finale de traitement cosmétique obtenue par le procédé de la présente invention est en général comprise entre 0,001 et 50 % en poids environ du poids total de la composition finale de traitement cosmétique, de préférence entre 0,005 et 30 %, et encore plus préférentiellement entre 0,01 et 20%.

Lorsque la composition cosmétique obtenue par le procédé de la présente invention est mélangée à un milieu cosmétiquement acceptable, le milieu est généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les alcools inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40% en poids par rapport au poids total de la composition finale, et encore plus préférentiellement entre 5 et 30% en poids.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que, par exemple, des huiles de silicone, des agents filmogènes, des céramides, des agents conservateurs, des agents opacifiants, mais aussi des huiles, des cires, des gommes, des pigments colorés ou nacrés.

Les additifs ci-dessus sont en général présents en quantité comprise pour chacun d'eux entre 0,01 et 20% en poids par rapport au poids de la composition finale.

Bien entendu, l'homme de l'art veillera à choisir ce ou ces éventuels composés de manière telle que les propriétés avantageuses attachées intrinsèquement à la composition cosmétique conforme à l'invention ne soient pas, ou substantiellement pas, altérées par la ou les adjonctions envisagées.

Le pH de la composition finale est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les sels alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 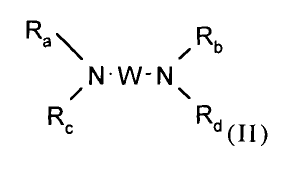 dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

La composition finale de traitement cosmétique peut se présenter sous des formes diverses, telles que sous forme de liquides, de crèmes, de gels ou sous toute autre forme appropriée pour réaliser un traitement des matières kératiniques, et notamment des fibres kératiniques, et de la peau.

La composition finale de traitement cosmétique peut être utilisée, par exemple, comme shampoing, après-shampoing, soin rincé ou non rincé, masque de soin profond, gel douche, lotion ou crème de traitement des fibres kératiniques.

L'invention a également pour objet un procédé de préparation d'une composition cosmétique pour le traitement cosmétique des fibres kératiniques, caractérisé en ce qu'il comprend une étape de percolation d'un fluide sous une pression d'au moins 3 bars au travers d'au moins un filtre solaire, sous forme solide ou pâteuse.

Elle a également pour objet les compositions obtenues par ce procédé, ainsi que leur utilisation pour la protection des fibres kératiniques contre les agressions extérieures. Les compositions sont de préférence exemptes d'agents conservateurs.

Elle a également pour objet l'utilisation cosmétique d'une composition obtenue par le procédé selon l'invention pour la fabrication d'une composition destinée à protéger les fibres kératiniques, et notamment la peau, contre les agressions extérieures.

Les compositions utilisées dans la présente invention peuvent être appliquées sur les fibres kératiniques par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

L'utilisation de filtres solaires permet de lutter notamment contre les radiations UV.

Les exemples ci-après sont destinés à illustrer la présente invention.

### Exemple 1

On mélange les ingrédients suivants dans les proportions indiquées en % en poids par rapport au poids total de mélange solide :
- mélange alcool cétylstéarylique/ alcool cétylstéarylique oxyéthyléné (30 OE) vendu sous la dénomination commerciale SINNOWAX AO par la société COGNIS 40 %
- acide 2-phényl-benzimidazol-5-sulfonique vendu sous la dénomination commerciale EUSOLEX 232 par la société MERCK 20 %
- chlorure de sodium 40 %

On place 5 g de ce mélange dans une machine expresso. On fait ensuite passer de la vapeur d'eau jusqu'à l'obtention d'une composition (A) présentant un volume final de 50 ml.

On peut ajouter ensuite à deux parties en poids de composition (A), une partie en poids d'une composition aqueuse (B) contenant 1 % en poids d'hydroxyéthylcellulose, pour faciliter l'application.

On obtient ainsi une composition de traitement cosmétique prête à être appliquée sur les cheveux.

On obtient des cheveux résistants aux agressions extérieures.

### Exemple 2

On mélange les ingrédients suivants dans les proportions indiquées en % en poids par rapport au poids total de mélange solide :
- mélange alcool cétylstéarylique/ alcool cétylstéarylique oxyéthyléné (30 OE) vendu sous la dénomination commerciale SINNOWAX AO par la société COGNIS 40 %
- acide 2-hydroxy-4-méthoxy-benzophénone-5-sulphonique vendu sous la dénomination commerciale UVINUL MS 40 par la société BASF 20 %
- inuline de racine de chicorée vendu sous la dénomination commerciale FRUTAFIT IQ par la société COSUN 40 %

On place 5 g de ce mélange dans une machine expresso. On fait ensuite passer de la vapeur d'eau jusqu'à l'obtention d'une composition (A) préséntant un volume final de 50 ml.

On peut ajouter ensuite à deux parties en poids de composition (A), une partie en poids d'une composition aqueuse (B) contenant 1 % en poids d'hydroxyéthylcellulose, pour faciliter l'application.

On obtient ainsi une composition de traitement cosmétique prête à être appliquée sur les cheveux.

On obtient des cheveux résistants aux agressions extérieures.

## Revendications

1. Utilisation cosmétique d'une composition obtenue par percolation d'un fluide sous une pression d'au moins 3 bars au travers d'au moins un agent protecteur des fibres kératiniques, sous forme solide ou pâteuse, pour la protection des fibres kératiniques contre les agressions extérieures.

2. Utilisation selon la revendication 1, **caractérisée en ce que** l'agent protecteur est choisi parmi les filtres solaires, les antioxydants, les agents anti-pollution et les agents anti-radicalaires.

3. Utilisation selon la revendication 2, **caractérisée en ce que** l'agent protecteur est choisi parmi les filtres solaires.

4. Utilisation selon la revendication 3, **caractérisée en ce que** les filtres solaires sont choisis parmi les anthranilates, les dérivés cinnamiques, les dérivés de dibenzoylméthane, les dérivés salicyliques, les dérivés du camphre, les dérivés de triazine, les dérivés de la benzophénone, les dérivés de β,β-diphénylacrylate, les dérivés de benzotriazole, les dérivés de benzalmalonate, les dérivés de benzimidazole, les imidazolines, les dérivés de bis-benzoazolyle, les dérivés de l'acide p-aminobenzoïque, les dérivés de méthylène bis-(hydroxyphényl benzotriazole), les dérivés de benzoxazole, les polymères filtres et silicones filtre, les dimères dérivés d'α-alkylstyrène, les 4,4-diarylbutadiènes, et leurs mélanges.

5. Utilisation selon la revendication 2, **caractérisée en ce que** les agents anti-pollution sont choisis parmi la vitamine C et ses dérivés, les phénols et polyphénols non colorants, l'épigallocatéchine et les extraits naturels en contenant, les extraits de feuille d'olivier, les extraits de thé, les anthocyanes, les extraits de romarin, les acides phénols, les stilbènes, les dérivés d'acides aminés soufrés, la N-acétylcystéine, les chélatants, les caroténoïdes et les dérivés indoles non colorants.

6. Utilisation selon la revendication 2, **caractérisée en ce que** vitamine E et ses dérivés, les flavonoïdes, les bioflavonoïdes, le coenzyme Q10 ou ubiquinone, la catalase, la superoxyde dismutase, la lactoperoxydase, le glutathion peroxydase et les quinones réductases, le glutathion, le benzylidène camphre, les benzylcyclanones, les naphtalénones substituées, les pidolates ; le phytantriol, le gamma-oryzanol, la guanosine, les lignanes, la mélatonine, les réductones et leurs dérivés dont l'acide érythorbique, l'acide ascorbique et le palmitate d'ascorbyle, le sulfite de sodium.

7. Utilisation selon l'une quelconque des revendications précédentes, **caractérisée en ce que** l'agent protecteur sous forme solide ou pâteuse est mis en oeuvre en mélange avec au moins un adjuvant.

8. Utilisation selon la revendication 7, **caractérisée en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono ou disaccharides, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, et les particules de poly(chlorure de vinylidène/acrylonitrile).

9. Utilisation selon la revendication 7 ou 8, **caractérisée en ce que** le ou les agents protecteurs sont présents en une quantité allant de 0,5 à 99% en poids, de préférence de 1 à 80 % et encore plus préférentiellement de 2 à 60% en poids par rapport au poids total agent(s) protecteur(s) et adjuvant.

10. Utilisation selon l'une quelconque des revendications précédentes, **caractérisée en ce que** l'étape de percolation est mise en oeuvre avec un fluide sous une pression comprise entre 3 et 30 bars, de préférence entre 10 et 30 bars.

11. Utilisation selon l'une quelconque des revendications précédentes, **caractérisée en ce que** l'étape de percolation est mise en oeuvre avec un fluide sous pression d'au moins 10 bars.

12. Utilisation selon l'une quelconque des revendications précédentes, **caractérisé en ce que** le fluide est de la vapeur d'eau, éventuellement accompagnée d'eau liquide.

13. Utilisation selon l'une quelconque des revendications 1 à 11, **caractérisé en ce que** le fluide est constitué par un ou plusieurs solvants organiques liquides et/ou gazeux cosmétiquement acceptables.

14. Utilisation selon l'une quelconque des revendications précédentes, **caractérisée en ce que** la composition est appliquée sur les fibres kératiniques.

15. Utilisation selon la revendication 14, **caractérisée en ce qu'**on applique la composition sur les fibres kératiniques par l'intermédiaire d'un dispositif ne nécessitant pas d'intervention humaine.

16. Utilisation selon la revendication 14, **caractérisée en ce que**, avant l'application, la composition est mélangée à un milieu cosmétiquement acceptable et/ou à un ou plusieurs additifs utilisés en cosmétique.

17. Utilisation selon l'une quelconque des revendications précédentes, **caractérisée en ce que** l'on prépare au moins deux compositions cosmétiques, on les mélange, et on applique le mélange sur les fibres kératiniques.

18. Utilisation selon l'une quelconque des revendications précédentes, **caractérisée en ce que** les agressions extérieures sont la pollution, le graissage, les radiations UV, la lumière, les radicaux libres ou le chlore.

19. Utilisation selon la revendication 18, **caractérisée en ce que** les agressions extérieures sont les radiations UV.

20. Utilisation selon l'une quelconque des revendications précédentes, **caractérisée en ce que** les fibres kératiniques sont les cheveux.

21. Procédé de préparation d'une composition cosmétique pour le traitement cosmétique des fibres kératiniques, **caractérisé en ce qu'**il comprend une étape de percolation d'un fluide sous une pression d'au moins 3 bars au travers d'au moins un filtre solaire, sous forme solide ou pâteuse.

22. Procédé selon la revendication 21, **caractérisé en ce que** le filtre solaire est choisi parmi les anthranilates, les dérivés cinnamiques, les dérivés de dibenzoylméthane, les dérivés salicyliques, les dérivés du camphre, les dérivés de triazine, les dérivés de la benzophénone, les dérivés de β,β-diphénylacrylate, les dérivés de benzotriazole, les dérivés de benzalmalonate, les dérivés de benzimidazole, les imidazolines, les dérivés de bis-benzoazolyle, les dérivés de l'acide p-aminobenzoïque, les dérivés de méthylène bis-(hydroxyphényl benzotriazole), les dérivés de benzoxazole, les polymères filtres et silicones filtre, les dimères dérivés d'α-alkylstyrène, les 4,4-diarylbutadiènes, et leurs mélanges.

23. Procédé selon la revendication 21 ou 22, **caractérisé en ce que** le filtre solaire sous forme solide ou pâteuse est mis en oeuvre en mélange avec au moins un adjuvant.

24. Procédé selon la revendication 23, **caractérisé en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono ou disaccharides, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, et les particules de poly(chlorure de vinylidène/acrylonitrile).

25. Procédé selon la revendication 23 ou 24, **caractérisé en ce que** le ou les agents protecteurs sont présents en une quantité allant de 0,5 à 99% en poids, de préférence de 1 à 80 % et encore plus préférentiellement de 2 à 60% en poids par rapport au poids total agent(s) protecteur(s) et adjuvant.

26. Procédé l'une quelconque des revendications 21 à 25, **caractérisé en ce que** l'étape de percolation est mise en oeuvre avec un fluide sous pression d'au moins 10 bars.

27. Procédé selon l'une quelconque des revendications 21 à 26, **caractérisé en ce que** le fluide est de la vapeur d'eau, éventuellement accompagnée d'eau liquide.

28. Procédé selon l'une quelconque des revendications 21 à 26, **caractérisé en ce que** le fluide est constitué par un ou plusieurs solvants organiques liquides et/ou gazeux cosmétiquement acceptables.

29. Composition susceptible d'être obtenue par le procédé selon l'une quelconque des revendications 21 à 28.

30. Composition selon la revendication 29, **caractérisée en ce qu'**elle est exempte d'agent conservateur.

31. Dispositif de conditionnement d'une composition selon la revendication 29 ou 30, comprenant un logement fermé délimité par au moins une paroi au moins en partie perméable à un fluide sous pression d'au moins 3 bars, la composition contenant au moins un filtre solaire sous forme solide ou pâteuse.

32. Dispositif selon la revendication 31, **caractérisé en ce que** le logement est délimité par deux feuilles scellées.

33. Dispositif selon la revendication 31, **caractérisé en ce que** le logement est délimité par une barquette fermée par un couvercle.
